# EUROPEAN PATENT SPECIFICATION

(11) **EP 1 627 623 B1**
(45) Date of publication and mention of the grant of the patent: **27.06.2007**
(21) Application number: 05015636.3
(22) Date of filing: 19.07.2005
(51) Int. Cl.: A61K 8/06, A61K 8/19, A61K 8/37, A61K 8/68, A61K 8/63, A61K 8/92, A61K 8/42, A61Q 19/00

(54) **Oil-in-water emulsified composition**
Oel-in-Wasser emulgierte Zusammensetzung
Composition emulsionné huile-dans-eau

(30) Priority: 20.07.2004 JP 2004211024
(43) Date of publication of application: 22.02.2006
(73) Proprietor: KAO CORPORATION, Tokyo 103-8210 (JP)
(72) Inventor: Kawakami, Kyoko Kao Corp. Research Lab., Tokyo 131-8501 (JP); Okada, Joji Kao Corp. Research Lab., Tokyo 131-8501 (JP)
(74) Representative: HOFFMANN EITLE

(56) References cited:
- EP-A- 1 486 194
- US-A1- 2002 022 611
- US-B1- 6 207 711
- PATENT ABSTRACTS OF JAPAN vol. 2003, no. 12, 5 December 2003 (2003-12-05) & JP 2005 002020 A (KAO CORP), 6 January 2005 (2005-01-06)
- PATENT ABSTRACTS OF JAPAN vol. 2003, no. 12, 5 December 2003 (2003-12-05) & JP 2005 002021 A (KAO CORP), 6 January 2005 (2005-01-06)
- PATENT ABSTRACTS OF JAPAN vol. 2003, no. 12, 5 December 2003 (2003-12-05) & JP 2005 002017 A (KAO CORP), 6 January 2005 (2005-01-06)

## Description

### FIELD OF THE INVENTION

The present invention relates to an oil-in-water emulsified composition containing sphingosines, cholesterols and liquefied ester oil.

### BACKGROUND OF THE INVENTION

Ester oil has been utilized as an oil phase component in an emulsified composition, which also acts to reduce greasiness of a product, or becomes an additive for improving miscibility of components contained in a formulation. Moreover, some act as a solubility improver for an active ingredient, colorant, flavor or the like; an agent which aids a long-lasting effect of these qualities; or a dispersing agent for inorganic powder. An external preparation for skin containing ester oil is generally known to be soft to skin and have excellent spreadability, and moreover, have good permeability and moisturizing effect. For the reasons described above, more ester oil has begun to be utilized instead of wax and hydrocarbon oil in recent years with an aim to improve feeling upon application of, for example, an external preparation for skin.

However, when ester oil is added to an emulsified composition, the surfactant will be dissolved in the ester oil, which will cause transition of surfactant from interface to oil phase. This will cause a disturbance on the interface, and consequently brings deterioration in the emulsified state. This will further result in deterioration in the external appearance as typified by loss of surface luster. Taking the abovementioned situation into consideration, if large amounts of surfactant are incorporated in attempting to achieve stable emulsification of ester oil, the outcome will be deterioration in feeling upon application as typified by stickiness and sliminess. For these reasons, stable incorporation of ester oil has been considered difficult.

In view of the foregoing, JP-A-2001-220315 discloses a cosmetic composition which is prepared without surfactant, in which oil phase containing polar oil is stably dispersed while maintaining a spherical shape, where the state is achieved by utilization of powdery component which has a characteristic of being dispersible in water but not in oil. The composition, however, contains lower alcohols such as ethanol at high concentration; therefore not applicable to non-alcoholic formulation. Further, a powdery feeling is unavoidable upon application for the powdery component contained in the product, and further, application procedure becomes complicated in that the product needs to be shaken before application since powdery component deposits while it is left to stand.

JP-A-2003-40731 discloses an emulsified composition which is highly safe to skin. For the preparation of the composition, polyoxybutylene polyglycerine monoalkyl (monoalkenyl) ether and polyglycerin monofatty acid ester are employed as hydrophilic surfactant, by which the polar oil is stably emulsified and moreover, production of aldehyde is inhibited. This, however, is not sufficient to reduce the amount of surfactant to be used.
As described above, it has been difficult to stably incorporate ester oil with an aim to improve the feeling upon application and the moisturizing effect.

### SUMMARY OF THE INVENTION

In the present invention, there is thus provided an oil-in-water emulsified cosmetic composition containing the following components (A), (B), (C) and (D);
(A) ester oil which is in liquid state at 25°C
(B) sphingosines represented by the formula (1)

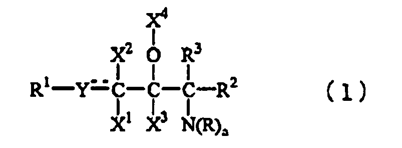

(wherein, R¹ represents a linear, branched or cyclic, saturated or unsaturated C₄₋₃₀ hydrocarbon group which may be substituted by a hydroxyl, carbonyl or amino group; Y represents a methylene group, a methine group or an oxygen atom; X¹,X² and X³ each independently represent a hydrogen atom, a hydroxyl group or an acetoxy groups, X⁴ represents a hydrogen atom, an acetyl group or a glyceryl group, or forms an oxo group together with the adjacent oxygen atom (with the proviso that when Y represents a methine group, either X¹ or X² represents a hydrogen atom and the other one does not exist and when X⁴ forms an oxo group, X³ does not exist); R² and R³ each independently represent a hydrogen atom, a hydroxyl group, a hydroxymethyl group or an acetoxymethyl group; R in number of "a" each independently represents a hydrogen atom or an amidino group, or a linear or branched, saturated or unsaturated hydrocarbon group having 1 to 8 carbon atoms in total and optionally having a substituent selected from hydroxyl, hydroxyalkoxy, alkoxy and acetoxy groups; "a" stands for 2 or 3; and a dashed line indicates a saturated bond or unsaturated bond)
(C) an acid compound selected from the group consisting of inorganic acid or an organic acid having 5 or less carbon atoms, and
(D) cholesterol or phytosterol,
(E) a diamide compound, which is
   represented by the formula (4); 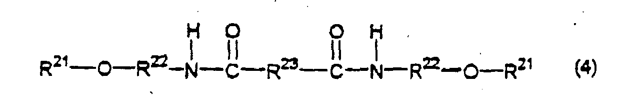 wherein, R²¹ each independently represents linear or branched C₁₋₂₂ hydrocarbon group which is unsubstituted or substituted by hydroxyl group and/or alkoxy group, R²² each independently represents linear or branched divalent C₁₋₁₂ hydrocarbon group, R²³ represents linear or branched divalent C₁₋₄₂ hydrocarbon group,
wherein a ratio of the liquid oil component containing component (A) in the total amount of oil phase is 5 to 90 wt%.

### DETAILED DESCRIPTION OF THE INVENTION

The present invention relates to an oil-in-water emulsified composition that can be stably emulsified despite incorporation of ester oil; that has excellent storage stability and external appearance; and that provides a superior feeling upon application.

The present inventors found that stable emulsification of a ceramide became possible by co-incorporation of sphingosines and acid compounds, which would otherwise be easily crystallized. This was made possible by a process of formation of a salt by neutralization between sphingosines and acid compounds, by which an amino group of sphingosines was cationized and consequently the sphingosines were enabled to behave like a surfactant. They further studied utilization of the compound for external preparations for skin.
The present invention was completed based on the following findings in the case where ester oil, which is a substance widely utilized as a component of an external preparation for skin, is used in combination; that oil-and-water interface can be more solidly stabilized by combining cationized sphingosines and cholesterol or phytosterol; and that liquefied ester oil can be stably emulsified by incorporating an oil component containing ester oil, which is in liquid state at 25°C, into the oil phase at a certain ratio.

oil-in-water emulsified composition of the present invention stably emulsifies ester oil, and maintains the stability over a long period of time without the aid from a surfactant. Further, it has a surface luster and looks good in exterior appearance, and also, provides a superior feeling upon application.

In oil-in-water emulsified composition of the present invention, the sphingosines represented by formula (1) and acid compounds selected from the group consisting of an inorganic acid and an organic acid having 5 or less carbon atoms undergo neutralization and form a salt, and sphingosines are cationized and exhibit a surfactant-like behavior. Further, cationized sphingosines stay in the oil-and-water interface by incorporation of cholesterol without dissolving or transferring into the oil phase, thereby surfactant-like-effects of cationized sphingosines are fully exerted. A stably emulsified composition is thought to be obtained from the above-described process. The composition maintains a favorably emulsified state, in which emulsified particles are uniformly dispersed. The composition is thereby furnished with a lustrous, beautiful appearance, and at the same time provided with superior storage stability. Moreover, thus obtained composition is stable without addition of a surfactant other than the cationized sphingosines, which means the amount of surfactant can be reduced compared with conventional emulsified preparations. Moreover, skin feels smoothly fit with the composition without greasiness or stickiness upon application, and at the same time, a high safety to the skin can be achieved.

The ester oil to be employed in the present invention which is in liquid state at 25°C (Composition A) possesses a mobility at 25°C under 1 atm. The ester oil of the present invention can be obtained by dehydration of acid containing carboxylic acid such as fatty acid, poly-basic acid, amino acid and a substance containing hydroxyl group as typified by alcohols such as lower alcohols, higher alcohols, poly alcohols or sterols such as cholesterol. In general, measurement of saponification value or ester value is adopted for determining if a substance is ester oil. In addition, ester oil purified from animals or plants and a specific ultraviolet absorbent are included in the ester oil.

The ester oil which is in liquid state at 25°C (Component A) includes monoester oil such as isopropyl myristate, butyl myristate, isopropyl palmitate, cetyl octanoate, isocetyl octanoate, isostearyl octanoate, cetostearyl octanoate, stearyl octanoate, isotridecyl myristate, isocetyl myristate, isostearyl myristate, isostearyl palmitate, isocetyl isostearate, isostearyl isostearate, hexyl isostearate, ethyl isostearate, isopropyl isostearate, octyldodecyl isostearate, ethyl oleate, ethyl linoleate, isopropyl linoleate, octyldodecyl myristate, octyl palmitate, octyl isopalmitate, octyl stearate, isocetyl stearate, butyl stearate, cetyl caprylate, decyl oleate, oleyl oleate, octyldodecyl oleate, hexyl laurate, isostearyl laurate, octyl isononanoate, isotridecyl isononanoate, octyldodecyl neopentanoate, hexyldecyl dimethyloctanoate, octyldodecyl dimethyloctanoate, octyl hydroxystearate, lauryl lactate, myristyl lactate, octyldodecyl lactate, octyldodecyl citrate, diglyceryl monoisostearate, alkyl benzoate, and cinnamic acid-ultraviolet absorbent such as 2-ethylhexyl paramethoxycinnamate; diester oil such as neopentylglycol dioctanoate, neopentylglycol dicaprate, di(phytosteryl/2-octyldodecyl) N-lauroyl-L-glutamate, di(cholesteryl/octyldodecyl) N-lauroyl-L-glutamate, ethyleneglycol dioctanoate, ethyleneglycol dioleate, propyleneglycol dioleate, propyleneglycol dicaprate, propyleneglycol di(caprylate/caprate), diglyceryl diisostearate, diisostearyl malate, glyceryl (isostearate/myristate), dioctyl succinate, diisobutyl adipate, dioctyl adipate, diethyl sebacate, diisopropyl sebacate, dioctyl sebacate and isocetyl 12-stearoylhydroxystearate; tri-ester oil such as glyceryl trioctanoate, trimethylolpropane trioctanoate, glyceryl tri(caprylate/caprate), glyceryl triisostearate, diglyceryl triisostearate, glyceryl tricaprylate, triethyl citrate, trioctyl citrate, triisocetyl citrate and trioctyldodecyi citrate; tetra-ester oil such as diglyceryl tetraoleate, acetyltriethyl citrate, acetyltributyl citrate, pentaerythritol tetraoctanoate, pentaerythritol tetraisostearate, diglyceryl tetraisostearate; a natural oil/fat containing mixture of monoester, diester and triester oil such as olive oil, rice bran oil, wheat germ oil, macadamia nut oil, avocado oil, almond oil, safflower oil, soy bean oil, corn oil, rapeseed oil, palm oil, mink oil and jojoba oil.

Among these, monoester oil, diester oil, tri-ester oil and natural oil/fat are preferred for better emulsification stability of the present invention. Further, one whose molecular weight lies within the range from 350 to 1000 or a natural oil/fat extracted from plants is preferred when the feeling upon application is taken into consideration. Yet further, isotridecyl myristate, octyldodecyl myristate, neopentylglycol dicaprate, diglyceryl diisostearate, glyceryl (isostearate/myristate), di(phytosteryl/2-octyldodecyl) N-lauroyl-L-glutamate, (cholesteryl/octyldodecyl) N-lauroyl-L-glutamate, macadamia nut oil, olive oil and jojoba oil are more preferred.

One or more kind of Component (A) can be used, and the content thereof in the oil-in-water emulsified composition of the present invention is preferably 0.05 to 70 wt%, more preferably 0.1 to 50 wt%, even more preferably 0.5 to 30 wt%.

In the oil phase, a liquid oil component other than Component (A) can be incorporated, and examples thereof include hydrocarbon oil such as liquid paraffin and squalane; silicone oil such as methyl polysiloxane, methyl cyclopolysiloxane, octamethyl trisiloxane, methyl phenylpolysiloxane, methylhydrogen polysiloxane, higher alcohol modified silicone oil, alkyl modified silicone oil, amino modified silicone oil; fluorine oil such as perfluoropolyether and perfluoroalkylether silicone.
The ratio of the liquid oil component containing component (A) in the total amount of oil phase of the emulsified composition of the present invention is 5 to 90 wt%, preferably 15 to 80 wt%, more preferably 35 to 65 wt%. A favorable appearance and superior emulsification stability can be obtained if the ratio lies within the above described range.
Additionally, the oil phase contains above described hydrocarbon oil, silicone oil, fluorine oil and the like in addition to the Components (A), (B), (D) and (E).

The sphingosines of the Component (B) of the present invention are the ones which are represented by the above described formula (1).
In the formula, R¹ represents a linear, branched or cyclic, saturated or unsaturated C₄₋₃₀ hydrocarbon group which may be substituted by a hydroxyl, carbonyl or amino group, preferably a linear, branched or cyclic, saturated or unsaturated C₇₋₂₂ hydrocarbon group which may be substituted by a hydroxyl group. More preferably, R¹ is a linear or branched C₁₀₋₂₀ alkyl group or a linear or branched C₁₀₋₂₀ alkyl group having, at a terminal thereof on the Y side, a hydroxyl group. When it is a branched alkyl group, it preferably has a methyl branched alkyl chain. Preferred examples thereof include tridecyl, tetradecyl, pentadecyl, hexadecyl, stearyl, 1-hydroxytridecyl, 1-hydroxypentadecyl, isohexadecyl and isostearyl groups.

Y represents any one of a methylene group (CH₂), a methine group (CH) and an oxygen atom.
X¹, X² and X³ each independently represent a hydrogen atom, hydroxyl group or an acetoxy group, X⁴ represents a hydrogen atom, an acetyl group, a glyceryl group or a substituent forming an oxo group together with the adjacent oxygen atom. Of these, preferred is the case where at most one of X¹, X² and X³ represents a hydroxyl group, the remaining ones represent a hydrogen atom, and X⁴ represents a hydrogen atom. When Y represents a methine group, either X¹ or X² represents a hydrogen atom and the other one does not exist. When X⁴ forms an oxo group, X³ does not exist.

R² and R³ each independently represent a hydrogen atom, a hydroxyl group, a hydroxymethyl group or an acetoxymethyl group. R³ is preferably a hydrogen atom.
The letter "a" stands for 2 or 3. When "a" stands for 2, R means R⁴ or R⁵ and when "a" stands for 3, R means R⁴, R⁵ or R⁶.

R⁴, R⁵ and R⁶ each independently represent a hydrogen atom or an amidino group, or a linear or branched, saturated or unsaturated hydrocarbon group having 1 to 8 carbon atoms in total and optionally having a substituent selected from hydroxyl, hydroxyalkoxy, alkoxy and acetoxy groups. As the hydroxyalkoxy group which may be a substituent for the hydrocarbon group, linear or branched C₁₋₇ hydroxyalkoxy groups are preferred. As the alkoxy group, linear or branched C₁₋₇ hydroxyalkoxy groups are preferred. Examples of R⁴, R⁵ and R⁶ include a hydrogen atom; linear or branched alkyl groups such as methyl, ethyl, propyl, 2-ethylhexyl and isopropyl; alkenyl groups such as vinyl and allyl; amidino groups; and hydrocarbon groups having 1 to 8 carbon atoms in total and having 1 to 6 substituents selected from hydroxyl group, hydroxyalkoxy group and alkoxy groups, such as hydroxymethyl, 2-hydroxyethyl, 1,1-dimethyl-2-hydroxyethyl, 2-hydroxypropyl, 2,3-dihydroxypropyl, 2-hydroxy-3-methoxypropyl, 2,3,4,5,6-pentahydroxyhexyl, 1,1-bis(hydroxymethyl)ethyl, 2-(2-hydroxyethoxy)ethyl, 2-methoxyethyl, 1-methyl-2-hydroxyethyl, 3-hydroxypropyl, 3-methoxypropyl, and 1,1-bis(hydroxymethyl)-2-hydroxyethyl.

Of these, a hydrogen atom, a methyl group, and an alkyl group which may be substituted by 1 to 3 substituents selected from hydroxyl group and hydroxyalkoxy groups, such as 2-hydroxyethyl, 1,1-dimethyl-2-hydroxyethyl, 1,1-bis(hydroxymethyl)ethyl, 2-(2-hydroxyethoxy)ethyl are more preferred.

As the sphingosines represented by formula (1), natural or natural type sphingosines, or derivatives thereof represented by the below-described formula (2) (which will hereinafter be described as "natural type sphingosine", collectively), or pseudo type sphingosines having a sphingosine structure represented by formula (3) (which will hereinafter be described as "pseudo type sphingosine") is preferred.
(I) Natural type sphingosine represented by formula (2):

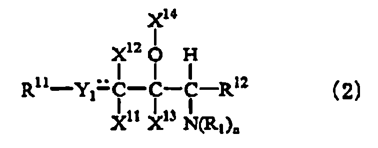

(wherein, R¹¹ represents a linear, branched or cyclic, saturated or unsaturated C₇₋₁₉ hydrocarbon group which may be substituted by a hydroxyl group; Y₁ represents a methylene or methine group; X¹¹, X¹² and X¹³ each independently represent a hydrogen atom, a hydroxyl group or an acetoxy group, X¹⁴ represents a hydrogen atom or forms an oxo group together with the adjacent oxygen atom (with the proviso that when Y₁ represents a methine group, either X¹¹ or X¹² represents a hydrogen atom and the other one does not exist, and when X¹⁴ forms an oxo group, X¹³ does not exist); R¹² represents a hydroxymethyl or acetoxymethyl group; R₁ in number of "a" each independently represents a hydrogen atom or an amidino group, or a linear or branched, saturated or unsaturated hydrocarbon group having 1 to 4 carbon atoms in total and optionally having a substituent selected from hydroxyl, hydroxyalkoxy, alkoxy and acetoxy groups; "a" stands for 2 or 3; and a dashed line indicates a saturated bond or unsaturated bond.

As R¹¹, linear, branched or cyclic, saturated or unsaturated C₇₋₁₉ hydrocarbon groups are preferred, with linear, saturated or unsaturated C₁₃₋₁₅ hydrocarbon groups being more preferred. It is preferred that "a" stands for 2 and R₁s each independently represent a hydrogen atom or a linear or branched C₁₋₄ alkyl group.

Specific examples of the natural type sphingosines represented by formula (2) include natural sphingosine, dihydrosphingosine, phytosphingosine, sphingadienine, dehydrosphingosine, dehydrophytosphingosine, and N-acetyl and N-alkyl derivatives (e.g., N-methyl derivatives) thereof.

As these sphingosines, natural (D(+) form) optically active derivatives, unnatural (L(-) form) optically active derivatives or a mixture thereof may be used. The relative configuration of these compounds may be any one of the configuration of a natural form, that of an unnatural form and that of their mixture.
Moreover, PHYTOSPHINGOSINE (listed in INCI; 8^{th} edition) and those represented by the below-described formulas are preferred.

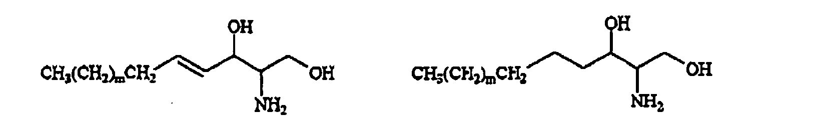

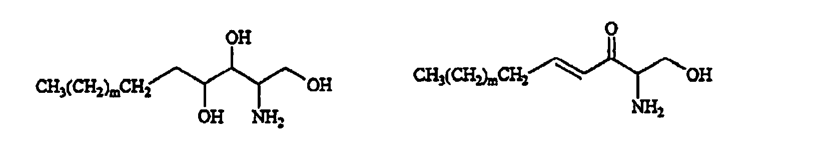

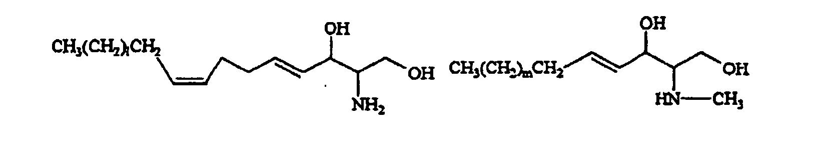

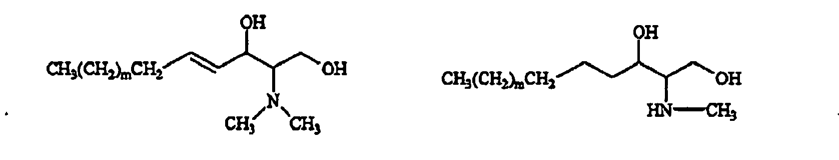

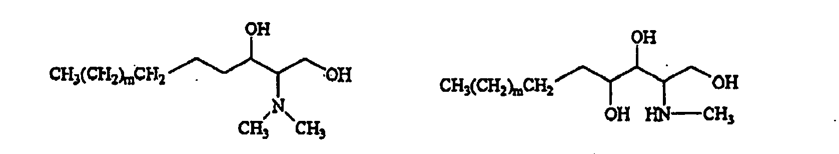

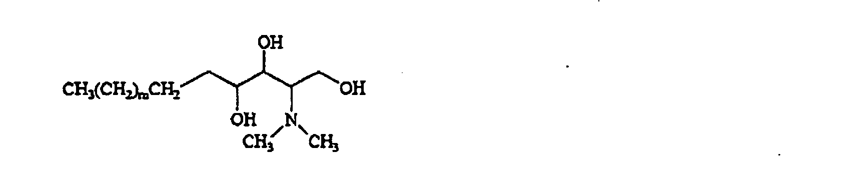

(wherein, "m" stands for 5 to 17 and "1" stands for 1 to 13.)

They may be an extract from natural source or a synthetic one, and commercially available one can also be used.
Examples of the commercially available natural type sphingosine include D-sphingosine (4-Sphingenine) (product of SIGMA-ALDRICH), DS-phytosphingosine (product of DOOSAN) and phytosphingosine (product of Cosmo-Pharm, Inc.).
(II) Pseudo type sphingosines represented by the following formula (3):

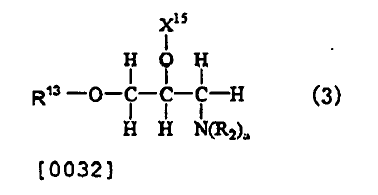

(wherein, R¹³ represents a linear, branched or cyclic, saturated or unsaturated C₁₀₋₂₂ hydrocarbon group which maybe substituted by a hydroxyl group; X¹⁵ represents a hydrogen atom, an acetyl group or a glyceryl group; R₂ in number of "a" each independently represents a hydrogen atom or an amidino group, or a linear or branched, saturated or unsaturated hydrocarbon group having 1 to 8 carbon atoms in total and optionally having a substituent selected from hydroxyl, hydroxyalkoxy, alkoxy and acetoxy groups, and "a" stands for 2 or 3.)

As R¹³, iso-branched alkyl groups having 14 to 20 carbon atoms are preferred, with an isostearyl group being more preferred. Still more preferred is an isostearyl group obtainable by using as raw material oil an isostearyl alcohol derived from a by-product of dimer acid preparation using a fatty acid derived from an animal or plant oil.

When "a" stands for 2, R₂ means R¹⁴ or R¹⁵, while when "a" stands for 3, R₂ means R¹⁴, R¹⁵ and R¹⁶.

Examples of R¹⁴, R¹⁵ or R¹⁶ include a hydrogen atom; linear or branched alkyl groups such as methyl, ethyl, propyl, 2-ethylhexyl and isopropyl; alkenyl groups such as vinyl and allyl; amidino group; alkyl groups having 1 to 8 carbon atoms in total and having a substituent selected from hydroxyl, hydroxyalkoxy and alkoxy groups, such as hydroxymethyl, 2-hydroxyethyl, 1,1-dimethyl-2-hydroxyethyl, 2-hydroxypropyl, 2,3-dihydroxypropyl, 2-hydroxy-3-methoxypropyl, 2,3,4,5,6-pentahydroxyhexyl, 1,1-bis(hydroxymethyl)ethyl, 2-(2-hydroxyethoxy)ethyl, 2-methoxyethyl, 1-methyl-2-hydroxyethyl, 3-hydroxypropyl, 3-methoxypropyl, and 1,1-bis(hydroxymethy)-2-hydroxyethyl.
Among these, a secondary amine having as either R¹⁴ or R¹⁵ a hydrogen atom and as the other one a 2-hydroxyethyl, 1,1-dimethyl-2-hydroxyethyl, 1,1-bis(hydroxymethyl)ethyl or 2-(2-hydroxyethoxy)ethyl group is preferred.

As the pseudo type sphingosine, the one having as R¹³ an isostearyl group, as X¹⁵ a hydrogen atom, as R¹⁴ a hydrogen atom, and as R¹⁵ an alkyl group having 1 to 3 substituent(s) selected from hydroxyl and hydroxyalkoxy groups, such as 2-hydroxyethyl, 1,1-bis(hydroxymethyl)ethyl, 1,1-dimethyl-2-hydroxyethyl or 2-(2-hydroxyethoxy)ethyl group is preferred.
The following pseudo type sphingosines (i) to (iv) are specific examples of the pseudo type sphingosine.

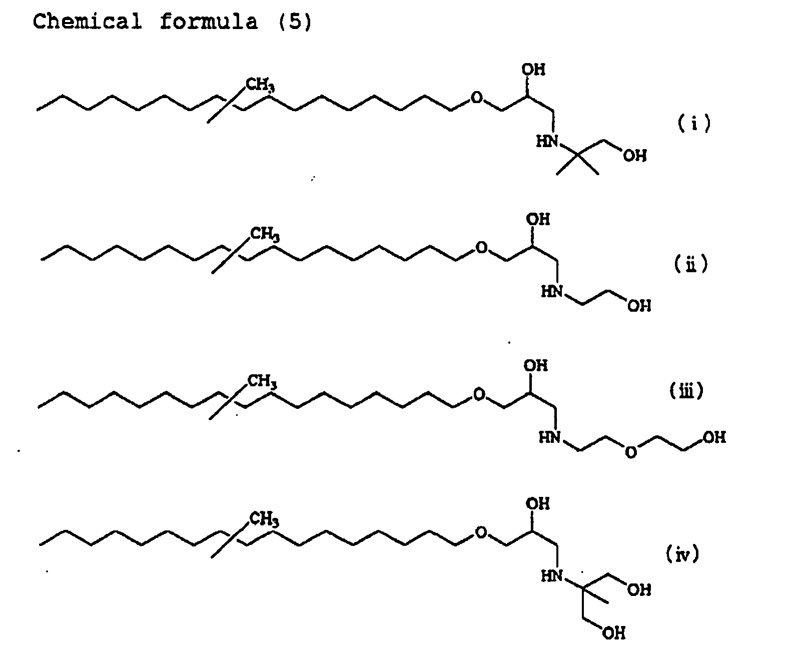

One or more kinds of Component (B) can be used, and the content thereof in the oil-in-water emulsified composition of the present invention is preferably 0.001 to 10 wt%, more preferably 0.005 to 5 wt%, even more preferably 0.01 to 3 wt%, for achieving better feeling upon application.

The acid compound (C) of the present invention selected from inorganic acids and organic acids having 5 or less carbon atoms is considered to form its salt with the amine group ot the sphingosine (B) by neutralization, and the cationized sphingosine acquire a surfactant-like activity. The presence of sphingosine salt can be examined by an infrared absorption spectroscopy or a proton nuclear magnetic resonance spectroscopy which has conventionally been used for identification of the structure of a compound.
As Component (C), the acid compound having pH of its 1 mol/L solution at 1 or greater but less than 7 at 25°C is preferred, with that having pH of from 1 to 6.5 being more preferred.

Examples of the inorganic acid as Component (C) include phosphoric acid, hydrochloric acid, nitric acid, sulfuric acid, perchloric acid and carbonic acid.
Examples of the organic acid include monocarboxylic acids such as formic acid, acetic acid, propionic acid, butyric acid, isobutyric acid, and valeric acid; dicarboxylic acids such as succinic acid, phthalic acid, fumaric acid, oxalic acid, malonic acid and glutaric acid; oxycarboxylic acids such as glycolic acid, citric acid, lactic acid, pyruvic acid, malic acid and tartaric acid; and amino acids such as glutamic acid and aspartic acid.

Among these, phosphoric acid, hydrochloric acid, succinic acid, citric acid, lactic acid, glutamic acid and aspartic acid are preferred for Component (C), in which lactic acid, glutamic acid and aspartic acid are more preferred.
One or more kind of Component (C) can be used. The content of Component (C) in the oil-in-water emulsified composition of the present invention is preferably from 0.001 to 10 wt%, more preferably from 0.005 to 5 %, even more preferably from 0.01 to 3 wt%.

Component (C) is preferably added in an amount of at least 0.3 moles, preferably 0.3 to 5 moles, more preferably 0.5 to 3 moles, per mole of component (B) in order to cationize the amine of the sphingosines (B). For example, it is preferred that an aqueous solution in which Component (C) and equimolar amount of Component (B) are mixed has pH of from 2 to 6 at 25°C (as measured by "HORIBA pH METER F-22" after correction with a phthalate standard solution).

The weight ratio of components (A), (B) and (C), which is denoted by the formula (A)/((B)+(C)), is preferably 0.1 to 100, more preferably 0.5 to 70, even more preferably 0.5 to 50, and still more preferably 2 to 50 for better stability.

Cholesterol or phytosterol as Component (D) of the present invention concentrates in the oil-water interface with sphingosines and acid compounds, thereby facilitating emulsification. The interface of oil phase and water phase is thus stabilized even under low or high temperature, and in this way emulsification stability is thought to be improved.

For Component (D), a combination of cholesterol and phytosterol can be used. The content of Component (D) in the oil-in-water emulsified composition of the present invention is preferably 0.05 to 20 wt%, more preferably 0.1 to 15 wt% and even more preferably 0.2 to 10 wt% for achieving superior stability and feeling upon application.
Further, the ratio of the Component (D) in the total amount of oil phase is preferably 0.1 to 80 wt%, more preferably 0.5 to 60 wt% and even more preferably 1 to 40 wt% for achieving better emulsification stability.

The weight ratio of component (B), (C) and (D), which is denoted by the formula ((B)+(C))/(D), is preferably 0.001 to 100, more preferably 0.005 to 50 and even more preferably 0.01 to 30 for better stability.
Also, the weight ratio of component (A) and (D), which is denoted by the formula (A)/(D), is preferably 0.05 to 70, more preferably 0.1 to 30 and even more preferably 0.1 to 20 for better stability.

The oil-in-water emulsified composition of the present invention contains the diamide compound represented by formula (4), which is disclosed in JP-A-2002-114666.

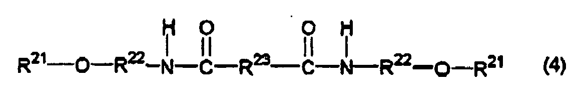

wherein, R²¹ each independently represents linear or branched C₁₋₂₂ hydrocarbon group which is unsubstituted or substituted by hydroxyl group and/or alkoxy group, R²² each independently represents linear or branched divalent C₁₋₁₂ hydrocarbon group, R²³ represents linear or branched divalent C₁₋₄₂ hydrocarbon group.

Addition of diamide compound improves the skin-protective effect (i.e., moisture retaining and suppression of water evaporation), thereby improving the feeling upon application of the product.

Component (E) is thought to further improve the emulsification stability by suppressing the mobility of the oil phase.
One or more kind of Component (E) may be used, and the content thereof in the oil-in-water composition of the present invention is preferably 0.01 to 40 wt%, more preferably 0.05 to 20 wt% and even more preferably 0.1 to 15 wt% for better emulsification stability and feeling upon application.

The water phase content in the total composition of the emulsified composition of the present invention is preferably 20 to 99 wt%, and more preferably 40 to 99 wt%. The water phase may contain water-soluble compositions other than water. Examples thereof include polyhydric alcohols such as 1,3-butylene glycol, propylene glycol, dipropylene glycol and glycerin; lower alcohols such as ethanol and isopropanol; humectants such as sorbitol, polyethylene glycol, glycine betaine, xylitol, trehalose, urea and amino acid; water soluble polymers such as xanthan gum, hydroxyethyl cellulose, methyl cellulose and hydroxypropyl guar gum; plant extracts such as eucalyptus extract, fucus extract, *Thujopsis dolabrata* extract, *Matricaria chamomilla* extract, Sanguisorba *officinalis* extract and tea extract; water-soluble medicinal agents such as magnesium ascorbyl phosphate salt, glycyrrhizate salt and vitamins; metal salts such as calcium chloride and magnesium phosphate; pH buffers such as citric acid, succinic acid, trisodium citrate; preservatives such as methylparaben, sodium dehydroacetate and phenoxyethanol.

The oil-in-water emulsified composition of the present invention may contain composition which is employed for conventional cosmetics and pharmaceutical products, in addition to above-described compositions. Example of such additional composition include organic powders such as cellulose powder, nylon powder, porous cellulose powder and porous nylon powder; silicone powder such as cross-linked silicone powder and cross-linked methylpolysiloxane; inorganic powders such as anhydrous silica, zinc oxide, titanium oxide and ferric oxide; algefacients such as menthol and camphor; UV ray protective agents; antioxidants; perfume; bactericides; and colorants.

It is not necessary to incorporate surfactant into the oil-in-water emulsified composition of the present invention for emulsification purpose; however, it may be incorporated for stabilization of active ingredients, dispersion of powdery ingredients or other purposes, if needed.

The emulsified composition of the present Invention may be prepared by the following exemplary procedure; in a first step, compositions (A), (B), (D), and (E), are melted for preparation of oil phase with heat above melting points. In a second step, an aqueous solution into which the composition (C) had been dissolved is gradually added to the oil phase for emulsification, while keeping the temperature constant.
The pH of the oil-in-water emulsified composition of the present invention is preferably 3 to 7, more preferably 4 to 6, when directly measured at 25°C.

The oil-in-water emulsified composition of the present invention may be used for cosmetics, pharmaceutical products, bath agents, bed-bathing agents, scalp care agents and the like. It is preferably used in, for example, basic skin care products such as lotion, emulsion and essence; makeup products such as basic cosmetics, foundation, control color, eye color and mascara; hair cosmetics such as hair cream and hair treatment agents.

### EXAMPLE

### Reference Example 1 to 7, Comparative Example 1 to 4

The oil-in-water emulsified composition having the composition shown in Table 1 was manufactured according to below-described procedure. An evaluation was made on the emulsified composition with respect to appearance, storage stability and feeling upon application. The pH or the composition was measured at 25°C following mixing the components. The results were summarized in Table 1.

### (Production method)

Following melting with heat the components (1) to (10) at a temperature ranging from 80 to 90 °C, a homogenous mixture of the component (11) to (14), which had been melted with heat at a temperature ranging from 80 to 90 °C, was added while stirring at 300r/min. The mixture was then mixed by a homomixer, and cooled to room temperature while stirring, thereby an oil-in-water emulsified composition was obtained.

### (Evaluation method)

### (1) Appearance:

Each composition was organoleptically evaluated by 10 expert panelists with respect to the surface luster and homogeneity. The evaluation standard was set as follows; 5 points for excellent "surface luster", 4 points for good, 3 points for fair, 2 points for little inferior, and 1 point for inferior. A composition was ranked A when the average score obtained by 10 panelists for the composition is 4.5 or more, and subsequently, a composition was ranked B for scoring 3.5 or more but less than 4.5, ranked C for scoring 2.5 or more but less than 3.5, and ranked D for scoring below 2.5.

### (2) Storage stability:

Each oil-in-water composition was filled in 50mL glass bottles and left to stand for three months at 40°C, 25°C and 5°C. Subsequently, the composition was observed visually and under microscope, and compared with the emulsification state of the composition right after preparation. The composition was evaluated according to the following standard.
A: No change was observed.
B: Subtle change was observed. (a particle aggregation was observed under the light microscope.)
C: Some change was observed. (slight oil floating, creaming or weak gelatinization was observed.)
D: Apparent change was observed. (oil separation, creaming, or gelatinization was observed.)

### (Feeling upon application)

Each oil-in-water emulsified composition was organoleptically evaluated by 10 expert panelists with respect to "smoothness upon spreading on the skin", "greaselessness upon application" and "how much skin feels stickiness-free after application", and ranked according to the following standard.
A: 9 or more panelists evaluated the composition excellent (good) .
B: 7 to 8 panelists evaluated the composition excellent (good) .
C: 4 to 6 panelists evaluated the composition excellent (good).
D: 3 or fewer panelists evaluated the composition excellent (good).

**Table I**

| Component (wt%) | | Reference Example | | | | | | | Comparative Example | | | |
|---|---|---|---|---|---|---|---|---|---|---|---|---|
| | | 1 | 2 | 3 | 4 | 5 | 6 | 7 | 1 | 2 | 3 | 4 |
| A | (1) Neopentylglycol dicaprate | 0.5 | 3 | 5 | 3 | | 2 | 1 | 3 | 3 | 0.05 | 3 |
| | (2) Octyldodecyl myristate | | | 5 | | 3 | | | | | | |
| B | (3) Pseudo-sphingosine*1 | | 0.04 | 0.2 | | 3 | 0.5 | 0.1 | | 0.6 | 1 | 0,2 |
| | (4) Phytosphingosine | 0.1 | | | 1 | 2 | | | | | | |
| D | (5) Cholesterol | 0.2 | 0.5 | 10 | 0.25 | | 1.5 | 1 | 0.5 | | 0.5 | 1 |
| | (6) Phytosterol | | 0.5 | | | 2 | | | 0.5 | | | |
| | (7) Vaseline | | | | 5 | | 2 | 5 | | | | |
| | (8) Stearyl alcohol | | | | 2 | | 1 | 2 | | | | |
| | (9) Squalane | | | | 5 | 2 | 1 | | 1 | 1 | | |
| | (10) Methylpolysiloxane (20cs) | | | 2 | 15 | 2 | 1 | | | | | 15 |
| C | (11) L-glutunic acid | 0.1 | 0.02 | | | 1.3 | 0.2 | 0.1 | | 0.3 | 0.4 | 0.1 |
| | (12) Lactic acid | | | 0.1 | 0.5 | 1 | | | | | | |
| | (13) Methylparaben | 0.2 | 0.2 | 0.2 | 0.2 | 0.2 | 0.2 | 0.2 | 0.2 | 0.2 | 0.2 | 0.2 |
| | (14) Purified water | Balance | | | | | | | | | | |
| Total | | 100 | 100 | 100 | 100 | 100 | 100 | 100 | 100 | 100 | 100 | 100 |
| Ratio of the liquid oil to the total oil phase (wt%) | | 63 | 74 | 54 | 74 | 50 | 44 | 11 | 80 | 87 | 3.2 | 94 |
| Ratio obtained by A/(B+C) | | 2.5 | 50 | 33 | 2 | 0.4 | 2.9 | 6.7 | - | 3.3 | 3.6 | 10 |
| Ratio of D in the total oil phase (wt%) | | 25 | 25 | 45 | 0.8 | 14 | 17 | 11 | 20 | 0 | 32 | 5.2 |
| pH | | 4 | 5 | 4 | 4 | 5 | 6 | 5 | 7 | 5 | 5 | 5 |
| Appearance | | A | B | B | C | A | A | A | D | C | B | C |
| Storage stability: 40oC, 3 months | | A | C | B | B | A | A | A | - | D | B | D |
| 25oC. 3 months | | A | B | B | B | A | A | A | - | - D | B | C |
| 5oC, 3 months | | A | B | C | B | A | A | A | - | C | C | C |
| Feeling upon application: smoothness upon spreading on the skin | | A | A | A | B | B | A | C | - | D | D | D |
| greaselessness upon application | | A | A | B | B | B | A | B | - | D | C | D |
| stickiness-free of the skin after application | | A | A | A | B | C | A | B | - | C | D | C |

| | | | | | | | | | | | | |
|---|---|---|---|---|---|---|---|---|---|---|---|---|
| *1; Sphingosine represented by formula (3), wherein R¹³ is isostearyl group, X¹⁵ is hydrogen, "a" is 2, R¹⁴ is 2-hydroxyethyl group and R¹⁵ is hydrogen. | | | | | | | | | | | | |

As apparent from the result in Table I, any one of the emulsified composition manufactured as in Examples 1 to 7 was excellent in external appearance and also exhibited high storage stability at 40°C, 25°C and 5°C, and moreover, provided superior feeling upon application. Oil failed to uniformly emulsify as early as just after preparation of the product in Comparative example 1, which lacks component (B) and (C). Comparative example 2, from which component (D) was omitted, exhibited unfavorable appearance for lacking surface luster, and was inferior in emulsification stability and feeling upon application. Comparative example 3, in which liquid oil component containing the component (A) occupies less than 5% of the total amount of oil phase, was inferior in emulsification stability in low temperature storage. It further brought stickiness and non-smoothness to the skin upon application. Comparative Example 4, in which liquid oil component occupies more than 90%, was inferior in appearance and emulsification stability, and further, brought greasy and sticky feeling to the skin upon application.

### Example 8 (lotion) (NOT FALLING UNDER THE SCOPE OF THE CLAIMS)

The lotion of the composition shown in Table II was manufactured according to the below-described method. The lotion thus prepared had pH of 4 and provided a favorable appearance, stability and feeling upon application.

### (Production method)

Following melting with heat the components (1) to (3) at 80 to 90 °C, a homogenous mixture of the component (4) to (12) and (14), which had been melted with heat at 80 to 90 °C was added while stirring at 300r/min. The mixture was then mixed by a homomixer, and cooled to 35°C while stirring, at which the composition (13) was added. A lotion was thus obtained.

**Table II**

| | Component | Wt% |
|---|---|---|
| B | (1) Phytosphingosine (product of Cosmo Pharm, Inc) | 0.2 |
| D | (2) Phytosterol (product of Tama Biochemical Co., Ltd.) | 0.2 |
| A | (3) Isotridecyl myristate | 0.5 |
| | (4) Glycerin | 5 |
| | (5) Trehalose | 1 |
| | (6) Dipropylene glycol | 3 |
| | (7) Hydroxypropyl guar gum | 1 |
| C | (8) L- aspartic acid | 0.1 |
| | (9) Ethylparaben | 0.1 |
| | (10) Althea extract | 0.1 |
| | (11) *Citrus* junos extract | 0.1 |
| | (12) *Thujopsis dolabrata* extract | 0.5 |
| | (13) Ethanol | 0.2 |
| | (14) Purified water | balance |

### Example 9 (Emulsion) (NOT FALLING UNDER THE SCOPE OF THE CLAIMS)

The emulsion of the composition of Table III was manufactured according to below-described method. The emulsion thus obtained had pH of 6, and provided a favorable appearance, stability and feeling upon application.

### (Production method)

Following melting with heat the components (1) to (9) at 80 to 90 °C, a homogenous mixture of the component (10) to (18), which had been melted with heat at 80 to 90 °C, was added while stirring at 300r/min. The mixture was then emulsified by a homomixer, and cooled to room temperature while stirring. An emulsion was thus obtained.

**Table III**

| | Component | Wt% |
|---|---|---|
| B | (1) Phytosphingosine (product of Cosmo Pharm, Inc.) | 0.4 |
| A | (2) glyceryl (isostearate/myristate) | 2 |
| D | (3) Phytosterol (product of Riken Vitamin, Co., Ltd.) | 1 |
| | (4) Methylpolysiloxane (10cs) | 10 |
| | (5) Phytosteryl isostearate | 2 |
| A | (6) Di (phytosteryl/2-octyldodecyl) N-lauroyl-L-glutamate | 3 |
| | (7) Cetyl alcohol | 1 |
| | (8) Perfluoropolyether*2 | 1 |
| | (9) Palmitic acid | 0.5 |
| | (10) glycerin | 10 |
| | (11) glycinebetaine (product of Asahi Kasei Corporation) | 1 |
| C | (12) Lactic acid | 0.1 |
| | (13) Hamamelis extract | 0.3 |
| | (14) Althea extract | 0.2 |
| | (15) Eucalyptus extract | 0.2 |
| | (16) Phellodendri Cortex extract | 0.1 |
| | (17) Methylparaben | 0.2 |
| | (18) Purified water | balance |

| | | |
|---|---|---|
| *2; FOMBLIN HC (product of Ausimont USA, Inc.) | | |

### Example 10 (Cream)

The cream of the composition of Table IV was manufactured according to below-described method. The cream thus obtained had pH of 6, and provided a favorable appearance, stability and feeling upon application.

### (Production method)

Following melting with heat the components (1) to (11) at 80 to 90 °C, a homogenous mixture of the component (12) to (22), which had been melted with heat at 80 to 90 °C, was added while stirring at 300r/min. The mixture was then mixed by a homomixer, and cooled to room temperature while stirring. A cream was thus obtained.

**Table IV**

| | Component | Wt% |
|---|---|---|
| B | (1) pseudo-sphingosine*1 | 0.2 |
| B | (2) Phytosphingosine (product of Cosmo Pharm Inc.) | 0.3 |
| A | (3) Diglyceryl diisostearate | 3 |
| A | (4) Olive oil | 5 |
| E | (5) Diamide compound*3 | 2 |
| | (6) Stearyl alcohol | 2 |
| D | (7) Phytosterol (Tama Biochemical Co., Ltd.) | 1 |
| D | (8) Cholesterol | 1 |
| | (9) Cholesteryl isostearate | 2 |
| | (10) Stearic acid | 0.3 |
| | (11) Methylpolysiloxane (50cs) | 5 |
| | (12) Glycerin | 15 |
| C | (13) L-glutamic acid | 0.2 |
| | (14) Polyethylene glycol 1540 (product of Sanyo Chemical Industries, Ltd.) | 3 |
| | (15) 1,3-butylene glycol | 2 |
| | (16) Phenoxyethanol | 2 |
| | (17) Methylparaben | 0.1 |
| | (18) Royal jelly extract | 0.3 |
| | (19) Fucus extract | 0.1 |
| | (20) *Thujopsis dolabrata* extract | 0.1 |
| | (21) Perfume | Trace |
| | (22) Purified water | balance |

| | | |
|---|---|---|
| *3; The compound disclosed in JP-A-2002-114666 | | |

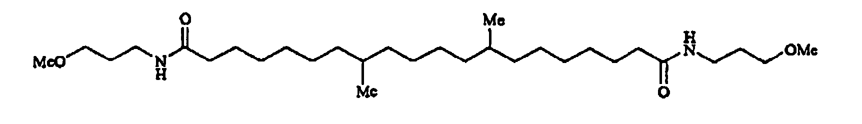

### Example 11 (Essence) (NOT FALLING UNDER THE SCOPE OF THE CLAIMS)

The essence of the composition of Table V was manufactured according to below-described method. The essence thus obtained had pH of 5, and provided a favorable appearance, stability and feeling upon application.

### (Production method)

Following melting with heat the components (1) to (9) at 80 to 90 °C, a homogenous mixture of the component (10) to (21), which had been melted with heat at 80 to 90 °C, was added while stirring at 300r/min. The mixture was then mixed by a homomixer, and cooled to room temperature while stirring. An essence was thus obtained.

**Table V**

| | Component | Wt% |
|---|---|---|
| B | (1) Pseudo-sphingosine*1 | 0.8 |
| A | (2) Macadamia nut oil | 4 |
| A | (3) Myristyl myristate | 2 |
| | (4) Tocopherol | 0.1 |
| | (5) Methylpolysiloxane(2cs) | 10 |
| A | (6) Jojoba oil | 3 |
| D | (7) Liquid paraffin | 1 |
| | (8) Phytosterol (product of Tama Biochemical Co., Ltd.) | 3 |
| | (9) β-carotene | Trace |
| | (10) Dipropylene glycol | 5 |
| | (11) Sodium hyaluronate | 0.2 |
| | (12) Hydroxypropyl methylcellulose*4 | 0.6 |
| | (13) Magnesium ascorbyl phosphate | 0.2 |
| C | (14) L-glutamic acid | 0.4 |
| | (15) Disodium edetate | 0.05 |
| | (16) Methylparaben | 0.2 |
| | (17) Green tea extract | 0.1 |
| | (18) *Sanguisorba officinalis* extract | 0.2 |
| | (19) *Thujopsis dolabrata* extract | 0.5 |
| | (20) *Matricaria chamomilla* extract | 0.5 |
| | (21) Purified water | balance |

| | | |
|---|---|---|
| *4; Metolose 60CH-10000 (product of Shin-etsu Chemical Co., Ltd.) | | |

### Example 12 (Sunscreen cream) (NOT FALLING UNDER THE SCOPE OF THE CLAIMS).

The sunscreen cream of the composition of Table VI was manufactured according to below-described method. The sunscreen cream thus obtained had pH of 5, and provided a favorable appearance, stability and feeling upon application.

### (Production method)

Following melting with heat the components (1) to (10) at 80 to 90 °C, a homogenous mixture of the component (11), (12), (14) to (16), which had been melted with heat at 80 to 90 °C, was added while stirring at 300r/min. The mixture was then mixed by a homomixer, and cooled to 35°C while stirring, at which component (13) was added. A sunscreen cream was thus obtained.

**Table VI**

| | Component | Wt% |
|---|---|---|
| B | (1) Pseudo-sphingosine *1 | 2 |
| | (2) Cholesteryl hydroxystearate | 2 |
| | (3) Methylcyclopolysiloxane (5-membered ring) | 15 |
| A | (4) 2-ethylhexyl p-methoxycinnamate | 5 |
| A | (5) Alkyl benzoic acid (C₁₂₋₁₅) | 2 |
| D | (6) Cholesterol | 2 |
| D | (7) Phytosterol (product of Tama Biochemical Co., Ltd.) | 1 |
| | (8) Beeswax | 3 |
| | (9) Behenyl alcohol | 3 |
| | (10) Methylpolysiloxane (10cs) | 5 |
| | (11) Dipotassium glycyrrhizate | 0.5 |
| | (12) Glycerin | 5 |
| | (13) Ethanol | 3 |
| | (14) Methylparaben | 0.2 |
| C | (15) L-glutamic acid | 1 |
| | (16) Purified water | balance |

## Claims

1. An oil-in-water emulsified cosmetic composition comprising the following components (A), (B), (C), (D) and (E):
(A) ester oil which is in liquid state at 25°C
(B) sphingosines represented by the formula (1) 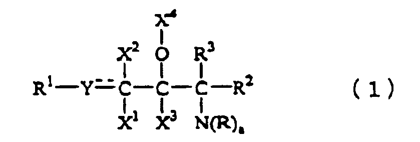 wherein, R¹ represents a linear, branched or cyclic, saturated or unsaturated C₄₋₃₀ hydrocarbon group which may be substituted by a hydroxyl, carbonyl or amino group; Y represents a methylene group, a methine group or an oxygen atom; X¹,X² and X³ each independently represent a hydrogen atom, a hydroxyl group or an acetoxy groups, X⁴ represents a hydrogen atom, an acetyl group or a glyceryl group or forms an oxo group together with the adjacent oxygen atom with the proviso that when Y represents a methine group, either X¹ or X² represents a hydrogen atom and the other one does not exist and when X⁴ forms an oxo group, X³ does not exist; R² and R³ each independently represent a hydrogen atom, a hydroxyl group, a hydroxymethyl group or an acetoxymethyl group; R in number of "a" each independently represents a hydrogen atom or an amidino group, or a linear or branched, saturated or unsaturated hydrocarbon group having 1 to 8 carbon atoms in total and optionally having a substituent selected from hydroxyl, hydroxyalkoxy, alkoxy and acetoxy groups; "a" stands for 2 or 3; and a dashed line indicates a saturated bond or unsaturated bond
(C) an acid compound selected from the group consisting of inorganic acid or an organic acid having 5 or less carbon atoms, and
(D) cholesterol or phytosterol,
(E) a diamide compound, which is represented by the formula (4); 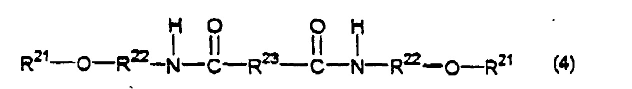 wherein, R²¹ each independently represents linear or branched C₁₋₂₂ hydrocarbon group which is unsubstituted or substituted by hydroxyl group and/or alkoxy group, R²² each independently represents linear or branched divalent C₁₋₁₂ hydrocarbon group, R²³ represents linear or branched divalent C₁₋₄₂ hydrocarbon group,
wherein a ratio of a liquid oil component containing component (A) in the total amount of oil phase is 5 to 90 wt%.

2. The oil-in-water emulsified composition of claim 1, wherein a weight ratio of the Components (A), (B) and (C) as denoted by a formula (A)/((B)+(C)) is 0.1 to 100.

3. The oil-in-water emulsified composition of claims 1 or 2, wherein a ratio of Component (D) in the total amount of oil phase is 0.1 to 80 wt%.

4. The oil-in-water emulsified composition of any one of claims 1 to 3, wherein the Component (A) is selected from a group consisting of monoester oil, diester oil, tri-ester oil and natural oil/fat.

## Patentansprüche

1. Emulgierte Öl-in-Wasser-Kosmetikzusammensetzung, umfassend die folgenden Komponenten (A), (B), (C), (D) und (E):
(A) Esteröl, das bei 25°C in flüssigem Zustand vorliegt,
(B) Sphingosine mit der Formel (1): 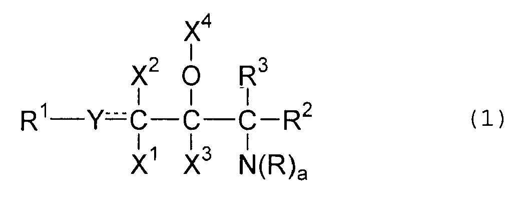 worin R¹ eine lineare, verzweigte oder cyclische, gesättigte oder ungesättigte C₄₋₃₀-Kohlenwasserstoffgruppe ist, die durch Hydroxyl-, Carbonyl- oder Aminogruppe substituiert sein kann; Y eine Methylen-, Methingruppe oder Sauerstoffatom ist; X¹, X² und X³ jeweils unabhängig ein Wasserstoffatom, eine Hydroxylgruppe oder Acetoxygruppen sind, X⁴ ein Wasserstoffatom, Acetylgruppe oder Glycerylgruppe ist oder eine Oxogruppe zusammen mit dem benachbarten Sauerstoffatom bildet, mit dem Vorbehalt, daß dann, wenn Y eine Methingruppe ist, X¹ oder X² ein Wasserstoffatom ist und das andere nicht existiert, und wenn X⁴ eine Oxogruppe ist, X³ nicht existiert; R² und R³ jeweils unabhängig ein Wasserstoffatom, eine Hydroxylgruppe, Hydroxymethylgruppe oder Acetoxymethylgruppe sind; R in der Anzahl von "a" jeweils unabhängig ein Wasserstoffatom oder eine Amidinogruppe oder eine lineare oder verzweigte, gesättigte oder ungesättigte Kohlenwasserstoffgruppe mit 1 bis 8 Kohlenstoffatomen insgesamt und gegebenenfalls mit einem Substituenten ist, ausgewählt aus Hydroxyl-, Hydroxyalkoxy-, Alkoxy- und Acetoxygruppen; "a" für 2 oder 3 steht und die gestrichelte Linie eine gesättigte oder ungesättigte Bindung anzeigt,
(C) eine Säureverbindung, ausgewählt aus der Gruppe bestehend aus einer anorganischen oder organischen Säure mit 5 oder weniger Kohlenstoffatomen, und
(D) Cholesterin oder Phytosterol,
(E) eine Diamidverbindung, die dargestellt ist durch die Formel (4): 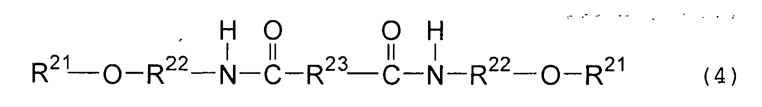 worin R²¹ jeweils unabhängig eine lineare oder verzweigte C₁₋₂₂-Kohlenwasserstoffgruppe ist, die unsubstituiert ist oder durch Hydroxylgruppe und/oder Alkoxygruppe substituiert ist, R²² jeweils unabhängig eine lineare oder verzweigte divalente C₁₋₁₂-Kohlenwasserstoffgruppe ist, R²³ eine lineare oder verzweigte divalente C₁₋₄₂-Kohlenwasserstoffgruppe ist,
worin das Verhältnis der flüssigen Ölkomponente, umfassend die Komponente (A), in der Gesamtmenge der Ölphase 5 bis 90 Gew.% ist.

2. Emulgierte Öl-in-Wasser-Zusammensetzung nach Anspruch 1, worin das Gewichtsverhältnis der Komponenten (A), (B) und (C), dargestellt durch die Formel (A)/((B)+(C) 0,1 bis 100 ist.

3. Emulgierte Öl-in-Wasser-Zusammensetzung nach Anspruch 1 oder 2, worin das Gewichtsverhältnis der Komponente (D) in der Gesamtmenge der Ölphase 0,1 bis 80 Gew.% ist.

4. Emulgierte Öl-in-Wasser-Zusammensetzung nach einem der Ansprüche 1 bis 3, worin die Komponente (A) ausgewählt ist aus der Gruppe bestehend aus Monoesteröl, Diesteröl, Triesteröl und natürlichem Öl/Fett.

## Revendications

1. Composition cosmétique émulsifiée huile dans l'eau comprenant les composants suivants (A), (B), (C), (D) et (E) :
(A) une huile d'ester qui se trouve à l'état liquide à 25 °C
(B) des sphingosines représentées par la formule (1) 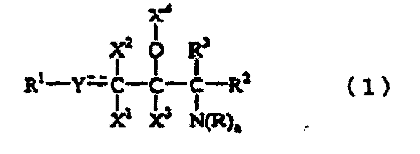 dans laquelle, R¹ représente un groupement hydrocarbure en C₄ à C₃₀ saturé ou insaturé, linéaire, ramifié ou cyclique qui peut être substitué par un groupement hydroxyle, carbonyle ou amino ; Y représente un groupement méthylène, un groupement méthine ou un atome d'oxygène ; X¹, X² et X³ représentent chacun indépendamment un atome d'hydrogène, un groupement hydroxyle ou des groupements acétoxy, X⁴ représente un atome d'hydrogène, un groupement acétyle ou un groupement glycéryle ou bien forme un groupement oxo en association avec l'atome d'oxygène adjacent à condition que quand Y représente un groupement méthine, soit X¹, soit X² représente un atome d'hydrogène 'et l'autre n'existe pas et quand X⁴ forme un groupement oxo, X³ n'existe pas ; R² et R³ représentent chacun indépendamment un atome d'hydrogène, un groupement hydroxyle, un groupement hydroxyméthyle ou un groupement acétoxyméthyle ; R au nombre de « a » représente chacun indépendamment un atome d'hydrogène ou un groupement amidino, ou bien un groupement hydrocarbure saturé ou insaturé, linéaire ou ramifié comportant de 1 à 8 atomes de carbone au total et comportant facultativement un substituant choisi parmi un groupement hydroxyle, hydroxyalcoxy, alcoxy et acétoxy ; « a » signifie 2 ou 3 ; et une ligne pointillée indique une liaison saturée ou une liaison insaturée
(C) un composé acide choisi parmi le groupe consistant en un acide minéral ou un acide organique comportant 5 atomes de carbone ou moins, et
(D) du cholestérol ou du phytostérol,
(E) un composé diamide, qui est représenté par la formule (4) ; 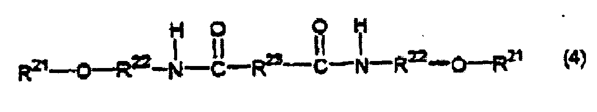 dans laquelle, R²¹ représente chacun indépendamment un groupement hydrocarbure en C₁ à C₂₂ linéaire ou ramifié qui est non substitué ou substitué par un groupement hydroxyle et/ou un groupement alcoxy, R²² représente chacun indépendamment un groupement hydrocarbure en C₁ à C₁₂ divalent linéaire ou ramifié, R²³ représente un groupement hydrocarbure en C₁ à C₄₂ divalent linéaire ou ramifié,
dans laquelle un rapport d'un composant huileux liquide contenant le composant (A) dans la quantité totale de la phase huileuse va de 5 à 90 % en poids.

2. Composition émulsifiée huile dans l'eau selon la revendication 1, dans laquelle un rapport en poids des composants (A), (B) et (C) tel que représenté par une formule (A) / ((B) + (C)) va de 0,1 à 100.

3. Composition émulsifiée huile dans l'eau selon les revendications 1 ou 2, dans laquelle un rapport du composant (D) dans la quantité totale de la phase huileuse va de 0,1 à 80 %.

4. Composition émulsifiée huile dans l'eau selon l'une quelconque des revendications 1 à 3, dans laquelle le composant (A) est choisi parmi le groupe consistant en une huile de monoester, une huile de diester, une huile de triester et une huile / graisse naturelle.
